# EUROPÄISCHE PATENTANMELDUNG

(11) **EP 1 025 872 A2**
(43) Veröffentlichungstag der Anmeldung: **09.08.2000**
(21) Anmeldenummer: 00101643.5
(22) Anmeldetag: 31.01.2000
(51) Int. Cl.: A61M 1/36, B04B 5/04, C12N 7/04

(54) **Schlauchset für einen Zellseparator zum Separieren von Blut in seine Komponenten sowie Zellseparator mit diesem Schlauchset**

(30) Priorität: 02.02.1999 DE 19904088
(71) Anmelder: Fresenius HemoCare GmbH, 61352 Bad Homburg v.d.H. (DE)
(72) Erfinder: Mitschulat, Heike, Dr., 66606 St. Wendel (DE); Klein, Sabine, 66903 Dittweiler (DE)
(74) Vertreter: Luderschmidt, Schüler & Partner GbR

(57) **Zusammenfassung**

Zur Zellseparation wird Blut eines Spenders einer Separationseinheit (1) zugeführt, in der mindestens eine der Komponenten des Bluts abgetrennt wird, die in einem Sammelbehältnis (24) gesammelt wird, wobei mindestens eine nicht abzutrennende Komponente wieder zurückgeführt wird. Eine oder mehrere der abgetrennte(n) und/oder nicht abzutrennende(n) Komponente(n) werden während der Zellseparation derart behandelt, daß Viren eliminiert oder die Infektiosität von Viren reduziert werden. Hierzu ist in eine Rückführleitung (8) des in den Zellseparator einzulegenden Schlauchsets eine Virenbehandlungseinheit (13) geschaltet. Ein zusätzliche Behandlung der Blutkomponenten nach der Zellseparation ist nicht erforderlich.

## Beschreibung

Die Erfindung betrifft ein Schlauchset für einen Zellseparator mit einem Schlauchsystem und einer Separationseinheit zur Trennung von Blut in seine Komponenten und ein Verfahren zum Separieren von Blut in seine Komponenten. Darüber hinaus bezieht sich die Erfindung auf einen Zellseparator mit einem derartigen Schlauchset.

Es sind verschiedene Vorrichtungen und Verfahren zur Gewinnung von Konzentraten bestehend aus bestimmten Blutkomponenten bekannt. Zur Gewinnung eines Thrombozytenkonzentrats beispielsweise wird Blut eines Spenders im extrakorporalen Kreislauf einer Zentrifugation unterworfen und in seine Bestandteile getrennt. Bei dieser Blutseparation erfolgt in einer ersten Stufe eine grobe Trennung des Blutes in zwei Phasen, nämlich in eine im wesentlichen aus Erythrozyten bestehende Fraktion und eine im wesentlichen aus thrombozytenreichem Blutplasma bestehende Fraktion. Zwischen diesen beiden Phasen befindet sich eine Anreicherung eines Leukozytengemischs. In einer zweiten Trennstufe wird das thrombozytenreiche Blutplasma dann in thrombozytenarmes Plasma und das gewünschte Thrombozytenkonzentrat separiert, das zur Behandlung von thrombozytopenischen Patienten dient. Die verbleibenden Blutkomponenten werden nach der Zentrifugation wieder vereint und dem Spender zugeführt. Eine Vorrichtung zur Durchführung eines derartigen Verfahrens ist beispielsweise aus der DE 42 27 695 A1 bekannt. Anstelle eines Thrombozytenkonzentrats kann mit der gleichen Vorrichtung aber auch ein Leukozytenkonzentrat aus der Zwischenphase gewonnen werden. Das Leukozytengemisch wird dabei angereichert und dann in Zyklen volumengesteuert aus der Kammer abgezogen.

Neben den Zellseparationsverfahren sind verschiedene Verfahren bekannt, mit denen sich Viren eliminieren oder die Infektiosität von Viren reduzieren lassen.

Die EP 0 110 409 A1 beschreibt ein Verfahren zur Adsorption von Viren in Blut. Ein Verfahren zur Inaktivierung von Viren in Blut ist aus der WO 91/03933 bekannt. Die Inaktivierung erfolgt dadurch, daß Blut mit Phenothiazinfarbstoffen versetzt und anschließend mit Licht bestrahlt wird. Eine photopheretische Behandlung für Zellkonzentrate zur Virusinaktivierung ist auch in Blood, Vol. 82, No. 1 (July 1), 1993: pp 292-297 beschrieben. Ein Eliminationsverfahren für Viren mittels einer Cellulosemembran ist aus dem Aufsatz Vox. Sang 1989; 56:230-236 bekannt.

Die Elimination bzw. Inaktivierung von Viren und die Gewinnung von Blutkomponenten durch Zentrifugation sind im Stand der Technik als getrennte Verfahren bekannt, obwohl es sich in der Praxis als sehr zeitaufwendig und wenig anwenderfreundlich erweist, wenn die jeweiligen Blutkomponenten zunächst gesammelt und erst dann einer entsprechenden Behandlung unterzogen werden. Dies erfordert mehr Bedienungspersonal und einen größeren Platzbedarf. Die Durchführung eines extrakorporalen Blutkreislaufs stellt für den Patienten eine hohe Kreislaufbelastung dar, zudem muß das Blut mit Antikoagulantien versetzt werden, wodurch auch das Gerinnungssystem stark belastet wird.

Der Erfindung liegt die Aufgabe zugrunde, ein Schlauchset für einen Zellseparator und einen Zellseparator mit einem derartigen Schlauchset zu schaffen, das die Gewinnung von einer Virenbehandlung unterzogenen Blutkomponenten ohne größeren Aufwand bei einer reduzierten Belastung für den Patienten erlaubt und ein vereinfachtes Verfahren anzugeben, daß die Gewinnung von einer Virenbehandlung unterzogenen Blutkomponenten bei einer reduzierten Belastung des Patienten ermöglicht.

Die Lösung dieser Aufgabe erfolgt erfindungsgemäß mit den Merkmalen des Patentanspruchs 1, 5 bzw. 9.

Eine reduzierte Belastung sowohl für das Kreislauf- als auch für das Gerinnungssystem des Patienten wird erfindungsgemäß dadurch errreicht, daß zwei extrakorporale Behandlungsverfahren in einem extrakorporalen Kreislauf durchgeführt werden. Die Virenbehandlung erfolgt also, bevor die Blutkomponente zurückgeführt oder gesammelt wird. Die Zellseparation und Virenbehandlung wird in einem Behandlungsschritt durchgeführt, wodurch die Separation der Blutkomponenten weniger zeitaufwendig und anwenderfreundlich wird.

In einer bevorzugten Ausführungsform weist die Behandlungseinheit einen Einlaß auf, der mit einem zu der Behandlungseinheit führenden Abschnitt der Sammel- oder Rückführleitung verbunden ist, und einen Auslaß auf, der mit einem von derselben abgehenden Abschnitt der Sammel- oder Rückführleitung verbunden ist. Das Schlauchsystem des Zellseparators bildet somit zusammen mit der Behandlungseinheit ein steriles Set, das einfach zu handhaben ist. Nach dem Gebrauch kann das Schlauchsystem mit der Behandlungseinheit einfach verworfen werden. Vorzugsweise dient die Behandlungseinheit zur Elimination von Viren oder Reduzierung der Infektiosität von Viren in der nicht abzutrennenden Komponente, insbesondere in dem Blutplasma, die über die Rückführleitung wieder zurückgeführt wird.

Die Behandlungseinheit kann mittels Konnektoren an das Schlauchsystem angeschlossen sein. Es ist aber auch möglich, daß die Behandlungseinheit einstückiger Bestandteil des Schlauchsystems ist. Die Konnektoren zum Anschluß der Behandlungseinheit können derart ausgebildet sein, daß die beiden Abschnitte der Sammel- oder Rückführleitung miteinander verbunden werden können, wenn die Behandlungseinheit nicht in die Sammel- oder Rückführleitung geschaltet ist. Um das Schlauchsystem auch ohne Behandlungseinheit betreiben zu können, kann die Behandlungseinheit auch mit einer Bypassleitung überbrückt sein, in der ein Sperrorgan, z. B. eine Schlauchklemme, zum Öffnen bzw. Schließen derselben angeordnet ist.

Zur Überwachung der Durchlässigkeit der Behandlungseinheit ist vorzugsweise eine Druckmeßeinheit vorgesehen, die den transmembranen Druck stromauf der Behandlungseinheit mißt.

Die Vorteile des erfindungsgemäßen Verfahrens kommen insbesondere in einem Zellseparator zum Tragen, bei dem in einer ersten Trennstufe Blut in eine vorwiegend aus Erythrozyten bestehende Fraktion und eine vorwiegend aus thrombozytenreichem Plasma bestehende Fraktion getrennt und in einer zweiten Trennstufe Leukozytenkonzentrat gewonnen wird. Die Behandlungseinheit zur Elimination von Viren oder Reduzierung der Infektiosität von Viren ist vorzugsweise der von der zweiten Trennstufe abgehenden Rückführleitung für das Blutplasma zugeordnet, das dem Spender wieder zugeführt wird.

Die gleichzeitige Abtrennung von Leukozyten ist insofern von Vorteil, als z. B. HIV, CMV und Hepatitisviren durch die Bestrahlung der Leukozyten inaktiviert werden können. Dadurch kann die Effektivität der Vireninaktivierung noch gesteigert werden, da neben der aktuten Virusinfektiositätsverminderung durch eine solche Bestrahlungsbehandlung auch die Virenreplikation in den Leukozyten verhindert und somit eine Art Langzeiteffekt erreicht werden kann.

Die Gewinnung von Plasma, das einer Behandlung zur Reduzierung der Virusaktivität, und einem Leukozytenkonzentrat, das einer Bestrahlung unterzogen werden kann, ist nicht nur in einer zweistufigen, sondern auch in einer einstufigen Separationseinheit durchführbar.

Im folgenden wird ein Ausführungsbeispiel des erfindungsgemäßen Zellseparators sowie ein Schlauchset für denselben unter Bezugnahme auf die Zeichnungen näher erläutert.

Es zeigen:
- Figur 1: die wesentlichen Komponenten eines Zellseparators zusammen mit dem zur einmaligen Verwendung bestimmten Schlauchset in schematischer Darstellung,
- Figur 2: eine Teilansicht eines alternativen Ausführungsbeispiels des Schlauchsets von Figur 1.
- Figur 3: eine Teilansicht eines weiteren Ausführungsbeispiels des Schlauchsets von Figur 1.

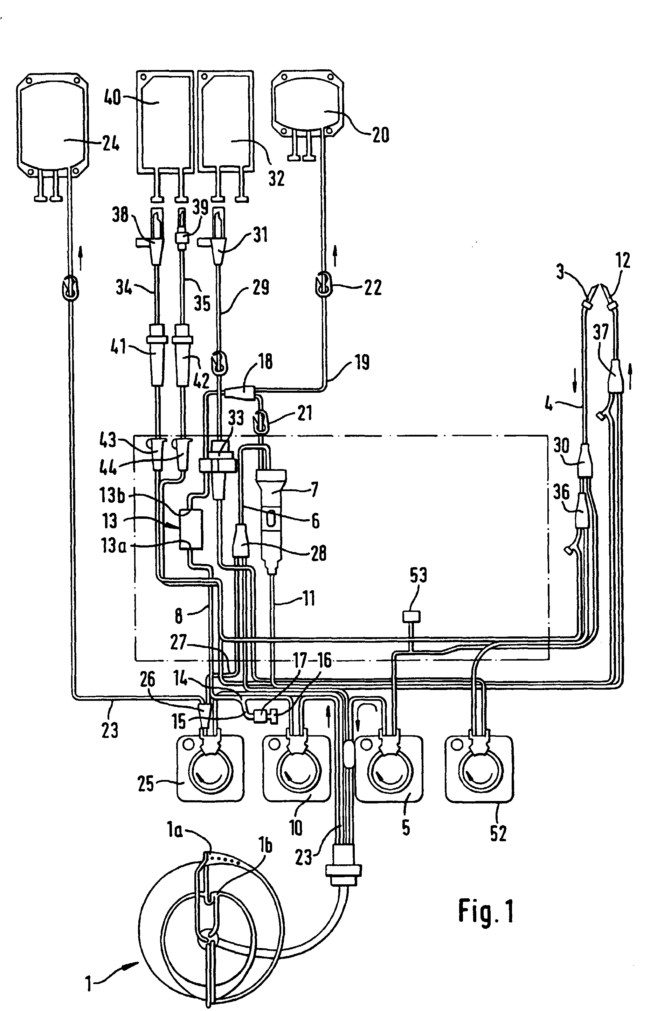

In Figur 1 sind nur die wesentlichen Komponenten des Zellseparators dargestellt, in den sich das zur einmaligen Verwendung bestimmte Schlauchset einlegen läßt. Da Vorrichtungen zum Separieren von Vollblut in seine Bestandteile zum Stand der Technik gehören, ist eine schematische Darstellung für das Verständnis der Erfindung ausreichend. Blutzellseparatoren sind beispielsweise aus den US-Patenten 3,655,123, 4,056,224 und 4,108,353 bekannt. Einrichtungen zur Überwachung der Phasengrenze in solchen Blutzentrifugen sind beispielsweise aus der EP 0 116 716 bekannt. Auf diese Offenbarung wird bei der nachfolgenden Beschreibung ausdrücklich bezug genommen.

In den Zellseparator wird ein zur einmaligen Verwendung bestimmtes Schlauchset eingelegt, das eine Separationseinheit 1, z. B. eine Separationskammer, und ein Schlauchsystem umfaßt. Die Separationseinheit 1 des Schlauchsets wird in eine nicht dargestellte Zentrifuge des Zellseparators eingelegt. Sie weist eine erste Trennstufe 1a auf, der das zu separierende Blut eines Spenders, das sogenannte Vollblut VB zugeführt wird. In dieser ersten Trennstufe wird das Vollblut grob in zwei Phasen getrennt. Die erste Fraktion besteht im wesentlichen aus Erythrozyten RBC, während die zweite Fraktion im wesentlichen aus Blutplasma besteht. Zwischen diesen beiden Phasen befindet sich eine Anreicherung eines Leukozytengemischs. Die Erythrozyten aus der ersten Trennstufe werden zurückgeführt. Die angesammelten Leukozyten werden periodisch in eine zweite Trennstufe 1b der Separationskammer 1 angehoben, um dann als Leukozytenkonzentrat abgetrennt zu werden, wobei Blutplasma sowie Restbestandteile aus der zweiten Trennstufe zurückgeführt und das gewonnene Leukozytenkonzentrat gesammelt werden.

Das Schlauchsystem weist einen arteriellen Vollblutanschluß 3, z. B. eine Nadel, auf, an der eine zu der ersten Trennstufe 1a der Separationskammer 1 führende Blutzuführleitung 4 angeschlossen ist. Die Blutzuführleitung 4 ist in eine peristaltische Vollblutpumpe 5 eingelegt, die Blut des Spenders ansaugt und in die Separationskammer fördert. Die Erythrozyten werden über eine Rückführleitung 6, die an die erste Trennstufe der Separationskammer angeschlossen ist, abgezogen. Die erste Rückführleitung führt zu einem ersten Einlaß eines Luftdetektors 7. Für die Förderung des Plasmas ist eine Sammel- oder Rückführleitung 8 vorgesehen, die einerseits an der zweiten Trennstufe 1b der Separationskammer 1 und andererseits an einem zweiten Einlaß des Luftdetektors 7 angeschlossen ist. Die Sammel- oder Rückführleitung 8 ist in eine peristaltische Plasmapumpe 10 eingelegt. Von einem Auslaß des Luftdetektors 7 führt eine gemeinsame Rückführleitung 11 zu einem venösen Vollblutanschluß 12, z. B. eine Nadel.

In die Sammel- oder Rückführleitung 8 ist stromab der Plasmapumpe 10 eine Behandlungseinheit 13 zur Elimination von Viren oder Reduzierung der Infektiosität von Viren geschaltet.

Die Behandlungeinheit weist einen Einlaß 13a, der mit dem zu der Behandlungseinheit führenden Abschnitt der Sammel- oder Rückführleitung verbunden ist und einen Auslaß 13b auf, der mit dem zu dem Luftdetektor 7 führenden Abschnitt der Sammel- oder Rückführleitung verbunden ist. Die Behandlungseinheit 13 kann unterschiedlich ausgebildet sein. Sie kann beispielsweise zur Elimination von Viren über eine Zellulosemembran verfügen, wie aus dem Aufsatz Vox. Sang 1989; 56:230-236 bekannt ist.

Zur Überwachung des Transmembrandrucks der Behandlungseinheit 13 zweigt über ein Y-Verbindungsstück 14 eine Schlauchleitung 15 mit einem Konnektor 16 zum Anschluß einer nicht dargestellten Druckmeßeinheit ab. Die Schlauchleitung 15 ist mit einem Sterilfilter 17 steril verschlossen.

Um das Blutplasma nicht dem Spender zurückzuführen, sondern sammeln zu können, zweigt von der Sammel- oder Rückführleitung 8 stromab der Behandlungseinheit 13 über ein Y-Verbindungsstück 18 eine Sammelleitung 19 ab, die zu einem Transferbeutel 20 führt. Je nachdem, ob Blutplasma gesammelt oder rückgeführt werden soll, wird eine dieser beiden Schlauchleitungen mittels einer Schlauchklemme 21, 22 abgeklemmt.

Von der zweiten Trennstufe 1b der Separationseinheit 1 geht eine Sammelleitung 23 für das gewonnene Leukozytenkonzentrat ab, die zu einem weiteren Transferbeutel 24 führt. Die Sammelleitung 23 ist in eine peristaltische Zellenpumpe 25 eingelegt, die das Leukozytenkonzentrat aus der zweiten Trennstufe abzieht und in den Transferbeutel fördert.

Über ein Y-Verbindungsstück 26 zweigt von der Sammelleitung 23 stromab der Zellenpumpe 25 eine weitere Rückführleitung 27 ab, die über ein Y-Verbindungsstück 28 stromauf des Luftdetektors 7 an die Rückführleitung 6 angeschlossen ist. Über die Rückführleitung 27 werden volumen-/zeitgesteuert Restblutbestandteile aus der zweiten Trennstufe der Separationseinheit abgezogen.

Das Schlauchsystem verfügt ferner über eine ACD-Leitung 29, die an einem Y-Verbindungstück 30 nahe des arteriellen Vollblutanschlusses 3 in die Blutzuführleitung 4 mündet und in eine ACD-Pumpe 52 eingelegt ist. Die ACD-Leitung 29 weist an ihrem Ende einen Konnektor 31 zum Anschluß an einen ACD-Beutel 32 auf. In die ACD-Leitung ist eine Tropfkammer 33 geschaltet.

Darüberhinaus verfügt das Schlauchsystem über zwei Spülflüssigkeitsleitungen 34, 35, die nahe des venösen bzw. arteriellen Vollblutanschlusses 3, 12 an Y-Verbindungensstücken 36, 37 in die Blutzuführleitung 4 bzw. gemeinsame Rückführleitung 11 münden. Die Spülflüssigkeitsleitungen 34, 35 weisen an ihren Enden Konnektoren 38, 39 zum Anschluß an einen Spülflüssigkeitsbeutel 40 auf. In die Spülflüssigkeitsleitungen sind Tropfkammern 41 bzw. 42 und Schlauchklemmen 43 bzw. 44 geschaltet. Zur Überwachung des arteriellen Drucks ist an der Blutzuführleitung stromauf der Vollblutpumpe ein Eingangsdruckmonitor 53 angeschlossen.

Das Schlauchsystem kann noch über weitere Komponenten beispielsweise Konnektoren, Sterilfilter oder Schlauchklemmen verfügen, die aber der besseren Übersichtlichkeit halber nicht dargestellt sind. So können beispielsweise die Transferbeutel 20, 24 nicht mit dem Schlauchsystem direkt verbunden, sondern über Konnektoren angeschlossen sein.

Da das aus der Separationskammer 1 abgezogene Plasma die Behandlungseinheit 13 durchströmt, werden in dem Plasma enthaltene Viren eliminiert oder die Infektiosität der Viren wird reduziert. Das Plasma kann dem Spender direkt zurückgeführt oder in dem Transferbeutel gesammelt werden. Weitere Maßnahmen zur Virenbehandlung nach Beendigung des Separationsverfahrens sind somit nicht erforderlich.

Das in dem Transferbeutel 24 gesammelte Leukozytenkonzentrat kann einer Bestrahlungsbehandlung zur Inaktivierung von Viren unterzogen werden, wie sie beispielsweise in Blood, Vol. 82, No. 1 (July), 1993:pp 292-297 beschrieben ist.

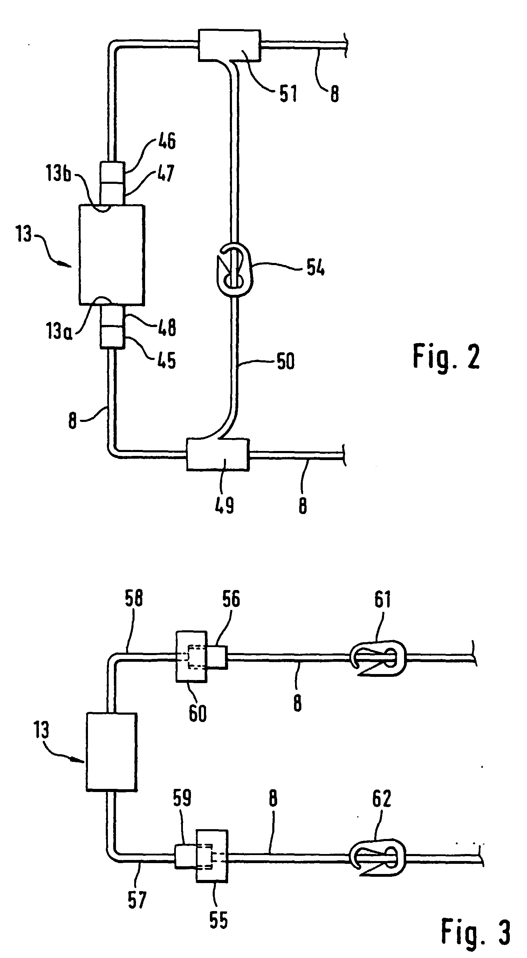

Figur 2 zeigt eine Teilansicht eines alternativen Ausführungsbeispiels des Schlauchsystems. Das Schlauchsystem von Figur 2 unterscheidet sich von dem Ausführungsbeispiel von Figur 1 dadurch, daß die Behandlungseinheit 13 nicht direkt mit der Sammel- oder Rückführleitung 8 verbunden, sondern über entsprechende Konnektoren angeschlossen ist. An dem zu der Behandlungseinheit 13 führenden Abschnitt der Leitung 8 und dem von der Behandlungseinheit wegführenden Abschnitt der Leitung sind Konnektoren 45, 46 angeschlossen, die mit entsprechenden Konnektoren 47, 48 am Einlaß 13a und Auslaß 13b der Behandlungseinheit verbunden werden. Von dem einen Abschnitt der Leitung 8 geht an einem Y-Verbindungsstück 49 eine Bypassleitung 50 ab, die zu einem Y-Verbindungsstück 51 an dem anderen Abschnitt der Leitung 18 führt. Zum Öffnen bzw. Schließen der Bypassleitung ist eine Schlauchklemme 54 vorgesehen.

Figur 3 zeigt eine Teilansicht eines weiteren Ausführungsbeispiels, bei dem die Behandlungseinheit 13 als Disposable ausbildet ist. Der zu der Behandlungseinheit führende Abschnitt der Leitung 8 weist einen männlichen und der zu dem Luftdetektor 7 führende Abschnitt der Leitung 8 weist einen weiblichen Luer-Lock-Konnektor 55, 56 auf, während an den Zu- und Abführleitungen 57, 58 der Behandlungseinheit 13 komplementäre Luer-Lock-Konnektoren 59, 60 vorgesehen sind. Wenn die Blutbehandlungseinheit 13 von dem Schlauchset abgetrennt wird, können die beiden Abschnitte der Leitung 8 mittels der Konnektoren 53, 54 miteinander verbunden werden. Zum Absperren der Leitungsabschnitte 8 sitzen auf den Schlauchenden Klemmen 61, 62.

## Patentansprüche

1. Schlauchset für einen Zellseparator, mit
- einer Separationseinheit (1) zur Trennung von Blut in seine Komponenten und
- einem Schlauchsystem mit
- einer zu der Separationseinheit führenden Blutzuführleitung (4) zum Zuführen von Blut,
- mindestens einer von der Separationseinheit abgehenden Sammelleitung (23) zum Abziehen einer abgetrennten Komponente,
- mindestens einer von der Separationseinheit abgehenden Rückführleitung (8) zum Rückführen einer nicht abzutrennenden Komponente,
gekennzeichnet durch,
eine Behandlungseinheit (13) zur Elimination von Viren oder Reduzierung der Infektiosität von Viren in einer oder mehreren der abgetrennten und/oder nicht abzutrennenden Komponente(n).

2. Schlauchset nach Anspruch 1, dadurch gekennzeichnet, daß die Behandlungseinheit (13) mit dem Schlauchset ein geschlossenes, einstückiges System bildet.

3. Schlauchset nach Anspruch 1 oder 2, dadurch gekennzeichnet, daß die Behandlungseinheit (13) einen Einlaß (13a) und einen Auslaß (13b) aufweist, wobei der Einlaß mit einem zu der Behandlungseinheit führenden Abschnitt der Sammel- oder Rückführleitung (8, 23) und der Auslaß mit einem von der Behandlungeinheit abgebenden Abschnitt der Sammel- oder Rückführleitung verbunden ist.

4. Schlauchset nach Anspruch 3, dadurch gekennzeichnet, daß an dem Schlauchsystem ein Anschluß (16) für eine den Transmembrandruck stromauf der Behandlungseinheit (13) messende Druckmeßeinheit vorgesehen ist.

5. Schlauchset nach einem der Ansprüche 1 bis 4, dadurch gekennzeichnet, daß die Separationseinheit (1) eine erste Trennstufe (1a) zur groben Trennung des Blutes in eine vorwiegend aus Erythrozyten bestehende Fraktion und eine vorwiegend aus thrombozytenreichem Plasma bestehende Fraktion und eine der ersten Trennstufe nachgeschaltete zweite Trennstufe (1b) zur Gewinnung eines Leukozytenkonzentrats aufweist, wobei von der ersten Trennstufe eine Rückführleitung (6) für die Erythrozytenfraktion und von der zweiten Trennstufe eine Sammelleitung (23) für Leukozytenkonzentrat und eine Rückführleitung (27) für Blutplasma abgeht, und daß die Behandlungseinheit (13) der von der zweiten Trennstufe abgehenden Rückführleitung (8) für Blutplasma zugeordnet ist.

6. Zellseparator mit einem Schlauchset, das eine Separationseinheit (1) zur Trennung von Blut in seine Komponenten und ein Schlauchsystem aufweist, wobei das Schlauchsystem aufweist:
- eine zu der Separationseinheit führende Blutzuführleitung (4) zum Zuführen von Blut,
- mindestens eine von der Separationseinheit abgehende und zu einem Sammelbehältnis (24) führende Sammelleitung (23) zum Abziehen einer abgetrennten Komponente und
- mindestens eine von der Separationseinheit abgehende Rückführleitung (8) zum Rückführen einer nicht abzutrennenden Komponente,
gekennzeichnet durch,
mindestens eine Behandlungseinheit (13) zur Elimination von Viren oder Reduzierung der Infektiosität von Viren in einer oder mehreren der abgetrennten und/oder nicht abzutrennenden Komponente(n).

7. Zellseparator nach Anspruch 6, dadurch gekennzeichnet, daß die Behandlungseinheit (13) mit dem Schlauchset ein geschlossenes, einstückiges System bildet.

8. Zellseparator nach Anspruch 6 oder 7, dadurch gekennzeichnet, daß die Behandlungseinheit (13) einen Einlaß (13a) und einen Auslaß (13b) aufweist, wobei der Einlaß mit einem zu der Behandlungseinheit führenden Abschnitt der Sammel- oder Rückführleitung (8) und der Auslaß mit einem von der Behandlungeinheit abgehenden Abschnitt der Sammel- oder Rückführleitung verbunden ist.

9. Zellseparator nach einem der Ansprüche 6 bis 8, dadurch gekennzeichnet, daß an dem Schlauchsystem ein Anschluß (16) für eine den Transmembrandruck stromauf der Behandlungseinheit (13) messende Druckmeßeinheit vorgesehen ist.

10. Zellseparator nach einem der Ansprüche 6 bis 9, dadurch gekennzeichnet, daß die Separationseinheit (1) eine erste Trennstufe (1a) zur groben Trennung des Blutes in eine vorwiegend aus Erythrozyten bestehende Fraktion und eine vorwiegend aus thrombozytenreichem Plasma bestehende Fraktion und eine der ersten Trennstufe nachgeschaltete zweite Trennstufe (1b) zur Gewinnung eines Leukozytenkonzentrats aufweist, wobei von der ersten Trennstufe eine Rückführleitung (6) für die Erythrozytenfraktion und von der zweiten Trennstufe eine Sammelleitung (23) für Leukozytenkonzentrat und eine Rückführleitung (27) für Blutplasma abgeht, und daß die Behandlungseinheit (13) der von der zweiten Trennstufe abgehenden Rückführleitung (8) für Blutplasma zugeordnet ist.

11. Verfahren zum Separieren von Blut in seine Komponenten, bei dem das Blut einer Separationseinheit zugeführt wird, in der mindestens eine der Komponenten des Blutes abgetrennt wird, die in einem Sammelbehältnis gesammelt wird, wobei mindestens eine nicht abzutrennende Komponente wieder zurückgeführt wird, dadurch gekennzeichnet, daß eine oder mehrere der abgetrennte(n) und/oder nicht abzutrennende(n) Komponente(n) derart behandelt werden, daß Viren eliminiert oder die Infektiosität von Viren reduziert werden.

12. Verfahren nach Anspruch 11, dadurch gekennzeichnet, daß das Blut in einer ersten Trennstufe der Separationseinheit in eine vorwiegend aus Erythrozyten bestehende Fraktion und eine vorwiegend aus thrombozytenreichem Plasma bestehende Fraktion getrennt wird, daß aus einer der ersten Trennstufe nachgeschalteten zweiten Trennstufe der Separationseinheit Leukozytenkonzentrat abgezogen und Blutplasma zurückgeführt wird, wobei das zurückgeführte Blutplasma zur Elimination von Viren oder Reduzierung der Infektiosität von Viren behandelt wird.

13. Verfahren nach Anspruch 12, dadurch gekennzeichnet, daß das aus der zweiten Trennstufe abgezogene Leukozytenkonzentrat zur Inaktivierung von Viren bestrahlt wird.
